(19) Europäisches Patentamt
European Patent Office
Office européen des brevets

(11) **EP 0 648 275 B1**

(12) **FASCICULE DE BREVET EUROPEEN**

(45) Date de publication et mention
de la délivrance du brevet:
**29.08.2007 Bulletin 2007/35**

(51) Int Cl.:
*C12Q 1/68* [(2006.01)]  *A61K 41/00* [(2006.01)]
*C12N 13/00* [(2006.01)]

(21) Numéro de dépôt: **93913082.9**

(22) Date de dépôt: **02.06.1993**

(86) Numéro de dépôt international:
**PCT/FR1993/000524**

(87) Numéro de publication internationale:
**WO 1993/024645 (09.12.1993 Gazette 1993/29)**

(54) **PROCEDE DE REGULATION EPIGENETIQUE DE LA BIOSYNTHESE DES PROTEINES PAR RESONANCE D'ECHELLE**

VERFAHREN ZUR EPIGENETISCHEN REGELUNG DER PROTEIN-BIOSYNTHESE DURCH WELLEN-RESONANZ

METHOD FOR THE EPIGENETIC REGULATION OF PROTEIN BIOSYNTHESIS BY SCALE RESONANCE

(84) Etats contractants désignés:
**AT BE CH DE DK ES GB GR IE IT LI LU NL PT SE**

(30) Priorité: **04.06.1992 FR 9206765**

(43) Date de publication de la demande:
**19.04.1995 Bulletin 1995/16**

(73) Titulaire: **STERNHEIMER, Joel**
**F-75005 Paris (FR)**

(72) Inventeur: **STERNHEIMER, Joel**
**F-75005 Paris (FR)**

(74) Mandataire: **Orès, Bernard et al**
**Cabinet ORES**
**36,rue de St Pétersbourg**
**75008 Paris (FR)**

(56) Documents cités:
**EP-A- 0 477 583        WO-A-84/03165**

• **CHEMICAL ABSTRACTS, vol. 106, no. 11, 16 Mars 1987, Columbus, Ohio, US; abstract no. 80530t, OHNO, SUSUMU ET AL. 'Repeats of base oligomers ( N = 3n +/- 1 or 2 ) as immortal coding sequences of the primeval world:construction of coding sequences is based upon the priciple of musical composition.' page 239 ;**

• **BIOLOGICAL ABSTRACTS vol. 90, no. 7 , 1 Octobre 1990, Philadelphia, PA, US; abstract no. 75110, BISTOLFI, F. 'A hydrogen-harps model for intracellular communication and its implications for the second genetic code.'**
• **CHEMICAL ABSTRACTS, vol. 110, no. 13, 27 Mars 1989, Columbus, Ohio, US; abstract no. 110189r, OHNO S. 'On periodicities governing the construction of genes and proteins.' page 270 ;**
• **CHEMICAL ABSTRACTS, vol. 116, no. 11, 16 Mars 1992, Columbus, Ohio, US; abstract no. 100618q, LI, W. ET AL. 'Long-range correlation and partial 1/f spectrum in a noncoding DNA sequence.' page 206 ;**
• **DATABASE WPIL Section Ch, Week 9146, Derwent Publications Ltd., London, GB; Class D13, AN 91-335582**

Remarques:
Le dossier contient des informations techniques présentées postérieurement au dépôt de la demande et ne figurant pas dans le présent fascicule.

**Description**

La présente invention est relative à un procédé de régulation épigénétique de la biosynthèse des protéines in situ, et aux applications de ce procédé, notamment dans les domaines de l'agro-alimentaire et de la santé. Il consiste à utiliser l'action régulatrice, par résonance d'échelle, sur la biosynthèse des protéines, des transpositions sonores de séquences temporelles de vibrations quantiques associées à leur élongation. Cette action peut être, soit une augmentation du taux de cette synthèse, en même temps qu'une régularisation de son rythme, soit une diminution de ce taux, suivant que la modulation des fréquences des vibrations utilisées est en phase ou en opposition de phase avec cette élongation (ceci étant vrai aussi bien pour les vibrations quantiques que pour leurs transpositions sonores). Le résultat obtenu est en outre stabilisé par l'action, toujours par résonance d'échelle, de transpositions lumineuses (colorées) des groupements de vibrations quantiques découlant de la conformation spatiale des protéines issues de cette élongation.

Ce procédé s'applique de façon spécifique à toute protéine dont on connaît la structure. Son emploi est toutefois d'autant plus approprié que la synthèse de cette protéine dépend plus fortement de facteurs épigénétiques, c'est-à-dire extérieurs à l'ADN de l'organisme auquel elle appartient, et spécialement dans le cas présent, de facteurs acoustiques et électromagnétiques; en outre il requiert, pour son application pratique, la détermination des agonismes et antagonismes métaboliques de ces protéines, dus aux phénomènes de résonance d'échelle associés naturellement à leur biosynthèse. La caractérisation de ces protéines dans leurs sous-ensembles métaboliques associés (délimitant ainsi leur rôle métabolique à partir de leur séquence en acides aminés) est encore un aspect de la présente invention.

L'identification des protéines propres à être régulées dans le cadre d'une application donnée inclut enfin d'autres critères -- tels que la correspondance entre des phénomènes acoustiques et électromagnétiques dont les effets peuvent être observés chez les êtres vivants eux-mêmes, et les séquences protéiques transposées -- qui constituent également une modalité de la présente invention.

I. La mise en évidence des propriétés musicales des particules élémentaires (J. Sternheimer, C.R. Acad. Sc. Paris <u>297,</u> 829, 1983), tout en soulignant la nécéssité d'une théorie cohérente pour ces dernières, a notamment permis d'y suggérer un rôle important pour l'échelle à laquelle se déroulent les phénomènes, comprise comme une dimension autonome par rapport à l'espace-temps. Des développements ultérieurs (amorcés dans J. Sternheimer, Colloque international "Louis de Broglie, Physicien et penseur", Ancienne Ecole Polytechnique, Paris, 5 - 6 novembre 1987) ont amené à conclure à l'existence physique d'ondes quantiques associées aux particules et se propageant, non seulement dans l'espace-temps mais aussi dans cette dimension d'échelle, reliant ainsi entre eux des niveaux successifs dans l'organisation de la matière. Ces ondes, dont nous avons pu écrire et résoudre les équations de propagation, autorisent ainsi une action d'une échelle sur l'autre entre phénomènes suffisamment semblables pour pouvoir constituer, en un sens mathématiquement bien défini, des harmoniques d'un même fondamental (J. Sternheimer, Ondes d'échelle, I. Partie physique, 1992, à paraître; II. Partie biologique, dont un résumé suit).

Les raisons théoriques de leur existence comme la conformité à l'expérience de diverses conséquences de leurs propriétés font apparaître les ondes d'échelle comme un phénomène universel dont la fonction est au départ d'assurer la cohérence entre les différentes échelles d'un système quantique, et qui prend notamment forme et peut être décrit dans le processus de biosynthèse des protéines. L'élongation de la chaîne peptidique résulte en effet de l'addition séquentielle d'acides aminés apportés sur le ribosome par des ARN de transfert (tARN) spécifiques. Lorsqu'un acide aminé, initialement à l'état libre, vient se fixer sur son tARN, il est à cet instant déjà suffisamment stabilisé vis-à-vis de l'agitation thermique, tout en conservant une relative autonomie du fait qu'il n'est lié au tARN que par un seul degré de liberté, pour que sa longueur d'onde broglienne atteigne l'ordre de grandeur de sa taille: cela lui confère des propriétés ondulatoires, et l'interférence entre l'onde d'échelle qui lui est alors associée et celles émises similairement par les autres acides aminés, résulte en une synchronisation, au bout d'un temps très bref que l'on peut évaluer à $10^{-12,5}$s environ, des fréquences propres associées à ces acides aminés suivant une même gamme musicale, qui dépend donc précisément de la population des ARN de transfert. Toutefois à l'approximation de la gamme tempérée, cette gamme est universelle, grâce à la distribution très particulière des masses de ces acides aminés, qui en est déjà très proche. (De façon similaire, les nucléotides de l'ADN sont eux aussi accordés sur une même gamme musicale, ainsi qu'on le vérifie aisément d'après leurs masses).

Mais le phénomène auquel nous nous référons va se manifester de façon plus explicite encore lorsque, l'acide aminé étant porté par son tARN, ce dernier vient à son tour se fixer sur le ribosome. A ce moment en effet, c'est-à-dire jusqu'au transfert qui va aller l'accrocher à la chaîne peptidique, la stabilisation vis-à-vis de l'agitation thermique devient telle que la longueur d'onde de l'acide aminé dépasse sa taille d'un bon ordre de grandeur. L'onde d'échelle qu'il émet alors va interférer, à l'échelle de la protéine, avec les ondes analogues préalablement émises par les autres acides aminés, ce qui va entraîner des contraintes de type musical pour la succession temporelle des fréquences propres associées à ces ondes, afin que les ondes d'échelle puissent (généralisant la situation précédente) poursuivre leur parcours et assurer ainsi une cohérence et une communication entre plusieurs niveaux de l'organisme -- par exemple, la seule succession de ces ondes aura déjà pour conséquence une minimisation des dissonnances (distances harmoniques) et écarts de fréquences (représentés par les distances mélodiques) entre acides aminés successifs; de plus, chaque onde d'échelle apparaissant comme une superposition d'ondes reliant deux niveaux donnés (et donc d'abord celui de chaque acide aminé à celui de la protéine) en des temps double, triple... du temps mis par la plus rapide, ceci impliquera l'existence de *périodes* de minimisation des distances harmoniques notamment, marquant des ponctuations dans la succession temporelle des fréquences; ce que les autres niveaux compléteront de corrélations d'autant plus riches et marquées qu'ils sont eux-mêmes plus nombreux à influer sur la synthèse de la protéine. Ceci a comme conséquence une prédiction remarquable: les protéines doivent posséder, dans la succession même des fréquences propres quantiques associées à la séquence de leurs acides aminés, des propriétés musicales d'autant plus nettes et élaborées que leur biosynthèse est plus sensible aux facteurs épigénétiques en général; et en retour, il doit être possible d'agir épigénétiquement, de façon spécifique pour chaque protéine, sur cette biosynthèse.

L'examen des séquences protéiques apparaissant dans la littérature [cf. M. O. Dayhoff, Atlas of protein sequence and structure, vol. 5 et suppl., N.B.R.F. (Washington) 1972-78; mises à jour accessibles via CITI 2, 45 rue des Saints-Pères, Paris] permet de confirmer qu'il en est bien ainsi; non seulement toutes les protéines possèdent des propriétés musicales dans l'enchaînement de leurs acides aminés, mais ces propriétés sont d'autant plus développées que ces protéines sont, d'une façon générale, plus sensibles épigénétiquement. En outre la *transposition acoustique des suites de fréquences propres* correspondant à la production d'ondes d'échelle en phase avec l'élongation d'une protéine donnée exerce une action *stimulante* sur la biosynthèse de cette protéine in vivo; et, de façon corrélative, une action *inhibante* au contraire pour des ondes d'échelle en opposition de phase. Ces actions, qui reproduisent à notre échelle les actions semblables s'exerçant déjà à l'échelle quantique entre protéines lors de leur synthèse Cet jouant donc un rôle important dans leur métabolisme: les protéines musicalement homologues sont ainsi, de façon systématique, métaboliquement agonistes), paraissent effectivement générales pour tous les êtres vivants sensibles aux vibrations sonores, et nous avons eu à maintes reprises l'occasion de les observer.

Dans le cas des animaux ayant un système nerveux, il semble que l'on puisse donner (au moins chez les vertébrés, où les "potentiels microphoniques" reproduisant fidèlement la forme d'onde appliquée ont bien été observés) la description suivante de ces phénomènes: l'onde sonore est transformée au départ du nerf auditif en impulsions électromagnétiques de même fréquence; celles-ci, en vertu de l'invariance d'échelle des équations des ondes d'échelle appliquées au photon (généralisant les équations de Maxwell), agissent alors directement, par résonance d'échelle, sur leurs transpositions quantiques; les amplitudes quantiques associées ayant leur carré proportionnel au nombre de protéines synthétisées simultanément, le phénomène de résonance va se traduire, dans le cas d'ondes d'échelle en phase, par une augmentation du taux en même temps qu'une régulation du rythme de la synthèse; et dans le cas d'ondes d'échelle en opposition de phase par une diminution de ce taux. [Ainsi qu'on pourra le noter, les potentiels microphoniques précédant, dans le nerf auditif, la naissance des influx nerveux proprement dits (cf. P. Buser et M. Imbert, Audition, Hermann éditeur, Paris 1987), le mécanisme invoqué ici ne sollicite pas, à ce stade, l'analyse cérébrale de ces influx]. Chez les plantes, la sensibilité (mécanique) aux sons est bien visible -- notamment par interférométrieet l'onde d'échelle fonctionne en théorie de façon analogue.

La solution de l'équation des ondes d'échelle, qui entraîne effectivement l'existence d'ondes d'échelle ayant une portée de l'ordre du nombre d'Avogadro (comme c'est le cas pour les transpositions mentionnées ci-dessus), amène à prévoir en outre des propriétés similaires pour les ondes d'échelle issues de la *répartition spatiale* des acides aminés (dont la longueur d'onde broglienne est alors du même ordre que leur taille) dans la protéine une fois synthétisée, avec une portée cette fois-ci de l'ordre de la racine carrée de ce nombre: l'examen de leurs structures tertiaires confirme l'existence d'harmonies de fréquences vibratoires entre acides aminés spatialement voisins dans les protéines (et particulièrement à leur surface, comme on peut s'attendre d'après leur longueur d'onde), en même temps que nous avons effectivement pu observer, à l'aide des transpositions *colorées* de ces fréquences, une stabilisation sensible des effets obtenus grâce aux transpositions musicales.

La présente invention découle de ces observations.

II. Pour le décodage des protéines, on procède ainsi:

1. On détermine la *suite des fréquences* de la façon suivante: à chaque acide aminé correspond une note de musique dont la fréquence exacte est obtenue, à partir des fréquences propres des acides aminés à l'état libre (proportionnelles à leurs masses), par minimisation de la distance harmonique globale $\Sigma_i P_j P_i P_j \log (\sup(p_i , q_j))$ calculée pour l'ensemble des couples de notes possibles, les $(p_i /q_j)$ étant les intervalles harmoniques globalement les plus proches des rapports de fréquences propres correspondants en tenant compte de leurs proportions respectives $P_i$, $P_j$ dans la population environnante des ARN de transfert tout en respectant la condition $\delta f < (\Delta f) /2$ où $\delta f$ est le déplacement de la fréquence initiale vers sa valeur synchronisée et $\Delta f$ l'écart entre les deux fréquences synchronisées successives de la gamme obtenue qui entourent cette fréquence initiale, puis (de façon analogue, au procédé décrit dans la demande de brevet français n° 83 02122 (FR-A-2 541 024) du même inventeur) transposition dans le domaine des fréquences audibles.

A l'approximation de la gamme tempérée, on obtient de cette façon un code universel pour la stimulation des synthèses protéiques:

Gly = la grave; Ala = do; Ser = mi; Pro, Val, Thr, Cys = fa; Leu, Ile, Asn, Asp = sol; Gln, Lys, Glu, Met = la; His = si bémol; Phe = si (ainsi que SeC); Arg, Tyr = do; Trp = ré aigu

et un autre pour son inhibition, obtenu à partir du précédent par symétrisation des logarithmes des fréquences autour de leur valeur centrale:

Trp = do; Arg, Tyr = ré; Phe, SeC = mi bémol; His = mi; Gln, Lys, Glu, Met = fa; Leu, Ile, Asn, Asp = sol; Pro, Val, Thr, Cys = la; Ser = si bémol; Ala = ré aigu; Gly = fa aigu de façon à résulter globalement en des ondes d'échelle respectivement en phase et en opposition de phase avec celles qui ont lieu dans le processus de synthèse. (Par code universel, nous entendons ici que ce code est identique pour toutes les protéines à l'approximation de la gamme tempérée; le la grave, pour une fréquence centrale située 76 octaves en-dessous du centre de gravité des fréquences initiales de la leucine, de l'isoleucine et de l'asparagine, est à 220 Hz. La définition de la distance harmonique donnée ci-dessus reprend, en l'explicitant, celle proposée par Y. Hellegouarch, C. R. Math. Rep. Acad. Sci. Canada, vol. 4, p. 277, 1982). Plus finement, les valeurs exactes dépendent des proportions des groupements d'acides aminés ci-dessus dans la population des ARN de transfert environnant la biosynthèse de la protéine, et peuvent être calculées à chaque fois.

2. On détermine la ou les *périodes* apparaissant dans la molécule.

L'existence même de ces périodes découle directement, comme nous l'avons signalé en I, de celle des ondes d'échelle. L'indication d'au moins certaines d'entre elles est usuellement donnée par la présence de *cadences* manifestes (telles que GG, F-S -- c'est-à-dire F suivi peu après de Sainsi que la cadence de fin du signal peptide lorsque celui-ci est présent, en stimulation; suites de R ou Y, en inhibition; exceptionnellement, pauses relatives impliquées par des variations de l'harmonie qui seraient autrement trop brutales; et dans tous les cas, cadences de retour sur la tonique) marquant des ponctuations du développement musical. On détermine ensuite plus précisément les *passages homologues* par la *répétition* soit directement de notes (lorsque c'est le cas, la période est donnée par un simple calcul d'autocorrélation des notes; ou encore plus finement -- en minimisant les écarts de notes -- par le nombre qui minimise la moyenne sur la protéine des distances mélodiques à un nombre entier de notes d'écart), soit de *mouvements mélodiques* (la période est alors donnée par un calcul d'autocorrélation des signatures ou signes des variations de fréquences d'une note à l'autre; ou plus finement, par un calcul d'autocorrélation des distances mélodiques d'une note à l'autre comptées avec leur signe, i. e. multipliées par les signatures correspondantes; ou plus finement encore, par le nombre qui minimise la moyenne sur la protéine des variations de proche en proche des distances mélodiques à un nombre entier de notes d'écart; la répétition des contours *mélodiques* étant de son côté précisée par un calcul d'autocorrélation des paires, ou mieux encore des triplets de signatures), soit encore par la logique du mouvement *harmonique* qui reproduit les notes ou le mouvement mélodique à une transposition harmonique simple près (octave, quarte ou quinte en général; la période est alors donnée par le nombre qui minimise la moyenne sur la protéine des distances harmoniques à un nombre entier de notes d'écart).

Quelquefois aussi, lorsqu'un "alignement" de séquences similaires -- notamment chez différentes espèces -- est disponible, la période apparaît dans les additions ou délétions entre certaines de ces séquences. Le résultat doit donner une progression cohérente mélodiquement et harmoniquement. On tient compte pour cela du fait que les *dernières notes* de chaque période ou membre de phrase -- d'une façon générale la seconde moitié, et plus particulièrement la dernière noteainsi que celles qui sont situées sur des *temps forts* (dont la caractérisation sera précisée au § 4) sont les plus importantes pour cette progression. Le résultat final est alors Le *plus significatif* (c'est-à-dire que l'on pondère ces différents éléments en fonction de leur importance relative dans la protéine, et notamment les distances harmonique et mélodique par le carré du rapport de leurs écarts-types normalisés) respectant l'ensemble de ces critères: il en existe généralement un, nettement plus significatif que les autres, comme cela se produit lorsque l'on cherche à déterminer par le calcul les repliements spatiaux des molécules; les cas analogues à l'allostérie existent cependant, et ont une signification biologique (stimulation ou inhibition par telle molécule ou par telle autre lors du métabolisme), mais portent alors plus fréquemment sur la position des barres de mesure que sur la période (fonction métabolique différente suivant le contexte par exemple CG-riche ou AT-riche, les barres de mesure dépendant de la composition de l'ADN comme les "arbres de Noël" visibles lors de certaines synthèses -- cf. B. Alberts et al., Biologie moléculaire de la cellule, 2è éd., trad. fr. Flammarion 1990, p. 539 - en témoignent).

3. On rectifie, le cas échéant, telle ou telle période particulière afin que les passages mélodiques en rapport (c'est-à-dire se répétant ou se suivant) se retrouvent à la même position dans la mesure: on en déduit les durées individuelles des notes. (Cette opération d'ajustement du phrasé à la mesure est comparable à ce qui se produit dans le phénomène bien connu de l'allongement des voyelles d'un texte chanté).

Dans la pratique, les opérations des § 2 et 3 s'effectuent le plus aisément à l'aide d'un clavier tel que ceux de marque Casio possédant une touche "one key play", ou d'un ordinateur programmé similairement à cet effet, dans lequel on a préalablement stocké en mémoire la suite des fréquences des notes telles que déterminées au §1, et où l'on fait ensuite défiler la séquence des notes, ce qui permet un contrôle et un ajustement de ces opérations. Celles-ci nécessitent toutefois alors quelques précautions; la prudence implique notamment de décoder aussi la même molécule ou une molécule musicalement homologue, dans le sens inhibiteur (ou en tout cas dans le sens inverse du sens initial), en tenant compte du fait que les molécules ont bien souvent un sens de décodage privilégié: il est fréquent en particulier, pour des couples de molécules exerçant sensiblement la même fonction, que l'une soit plus musicale en inhibition et l'autre en stimulation (c'est notamment le cas dans le métabolisme de l'immunité et de l'auto-immunité); dans ce cas, la présence et la distribution des cadences (qui diffèrent en stimulation et en inhibition, cf. § 1) permet normalement de les reconnaître d'emblée, et de se préserver en conséquence.

4. On vérifie le style rythmique par la distribution des bases de l'ADN: d'abord, le cas échéant, par leurs autocorrélations (lorsque, la molécule étant suffisamment musicale, la période de ces autocorrélations correspond à celle de la protéine; elles déterminent alors en principe les barres de mesure, les rangs des triplets de bases - ou plus exactement des bases en troisième position dans ces triplets - pour lesquels les pics d'autocorrélation sont les plus élevés correspondant aux notes les plus accentuées) puis en utilisant l'usage des codons, par comparaison avec des molécules connues (déjà décodées, ou plus régulières et donc posant moins de difficultés) ayant le même style rythmique supposé: le style de rythme musical (qui, en contraignant l'accentuation des notes, influe ainsi sur le choix des bases en troisième position) déterminant (au moins approximativement) de façon univoque cet usage des codons, des molécules de même style doivent donc avoir (très sensiblement) le même usage des codons. On rectifie le cas échéant corrélativement le décodage de certains passages.

5. On cherche alors à déterminer le timbre. Celui-ci est en principe distinct pour chaque molécule, et en tout cas pour chaque répartition des notes. En théorie il dépend principalement de la molécule elle-même, mais il dépend aussi de chacun des niveaux de l'organisme, qui rétroagissent sur la structure harmonique des acides aminés. Une première approche en est fournie par l'ajustement de la répartition des notes de la molécule à la courbe théorique de cette répartition (telle qu'elle peut se déduire de l'équation des ondes d'échelle, et qui correspond à ce que l'on observe en moyenne sur l'ensemble des protéines), d'où l'on déduit (comme dans la demande de brevet français n° 83 02122 (FR-A-2 541 024)) quels harmoniques sont amplifiés et lesquels sont atténués dans le timbre recherché; on sélectionne alors le timbre le plus approchant dans une palette de timbres naturels donnés à priori (telle qu'une mémoire de voix pour échantillonneurs, ou comme on en trouve déjà incluses dans de nombreux expandeurs et logiciels musicaux). Ceci implique plus précisément de distinguer trois situations: répartition des notes constante le long de la molécule (on a alors une structure harmonique relativement fixe); variations brutales de répartition (on a alors plusieurs timbres d'instruments successifs, par exemple cytochrome C avec plusieurs registres d'orgue); variations progressives de répartition (celle-ci reproduit alors l'évolution de la structure harmonique d'une seule note dans le temps, par exemple myosine où cette évolution indique très nettement un timbre de trompette).

D'autre part, la détermination du tempo ne pose en principe pas de réel problème au technicien, dans la mesure où il découle normalement du style rythmique déterminé plus haut; il est en général d'autant plus rapide qu'il y a d'importantes redondances dans la séquence protéique, comme c'est notamment le cas pour les protéines fibreuses.

6. On détermine enfin les couleurs par application du code, lui aussi universel en première approximation, déduit des fréquences de vibration des acides aminés individuels à l'aide de la formule (issue de la théorie des ondes d'échelle) $\nu \simeq \nu_o$ Arg ch($e^{(f/f_o)\text{Logch1}}$), où $f$, $f_o$ représentent les fréquences propres quantiques associées aux acides aminés comme précédemment et $\nu$, $\nu_o$ celles des couleurs, les indices dénotant les valeurs centrales; ce qui fournit le code suivant relatif à la stabilisation des protéines synthétisées in situ (celui relatif à la stabilisation de leur inhibition s'en déduisant en associant aux acides aminés les couleurs complémentaires):

Gly = rouge sombre; Ala = vermillon; Ser = orange; Pro, Val, Thr, Cys = ocre; Leu, Ile, Asn, Asp = citron; Gln, Glu, Lys, Met = vert; His = émeraude; Phe = bleu; Arg, Tyr = indigo; Trp = violet,

ces fréquences étant ensuite décalées vers le rouge ou le violet en fonction de la répartition globale des fréquences de la molécule, de façon analogue au timbre précédemment. Les positions spatiales des couleurs étant alors celles qu'occupent les acides aminés dans les représentations spatiales tridimensionnelles des molécules.

III. Exemples.

On trouvera ci-après quelques exemples de décodages musicaux et colorés de séquences protéiques. (Dans ces exemples comme dans les figures, nous utiliserons par commodité les codes à une lettre pour les acides aminés, soit Gly = G; Ala = A; Ser = S; Pro, Val, Thr, Cys = P, V, T, C respectivement; Leu, Ile, Asn, Asp = L, I, N, D; Gln, Glu, Lys, Met = Q, E, K, M; His = H; Phe = F; Arg, Tyr = R, Y; Trp = W).

1) *Exemple de protéine régulière de bout en bout.* Sur les alignements évolutifs d'une protéine particulièrement bien étudiée, le cytochrome C, on remarque une délétion constante de huit acides aminés (quelquefois sept) chez les protéines animales par rapport aux protéines végétales. L'examen des autocorrélations de notes et de contours mélodiques confirme cette première indication de valeur de la période musicale; si l'on compte en effet le nombre de fois que l'on rencontre la même note, ainsi que le même sens de variation des hauteurs de notes trois fois de suite (le même triplet de signatures), à un nombre entier k de notes de distance, on obtient le résultat suivant:

| Valeurs de k | 1 | 2 | 3 | 4 | 5 | 6 | 7 | 8 | 9 | 10 | 11 | 12 |
|---|---|---|---|---|---|---|---|---|---|---|---|---|
| Autocorrélations de notes | 19 | 15 | 15 | 20 | 19 | 15 | 17 | 21 | 14 | 17 | 18 | 13 |
| Autocor. de contours mél. | 1 | 7 | 4 | 6 | 5 | 10 | 8 | 13 | 5 | 4 | 4 | 4 |
| Total | 20 | 22 | 19 | 26 | 24 | 25 | 25 | <u>34</u> | 19 | 21 | 22 | 17 |

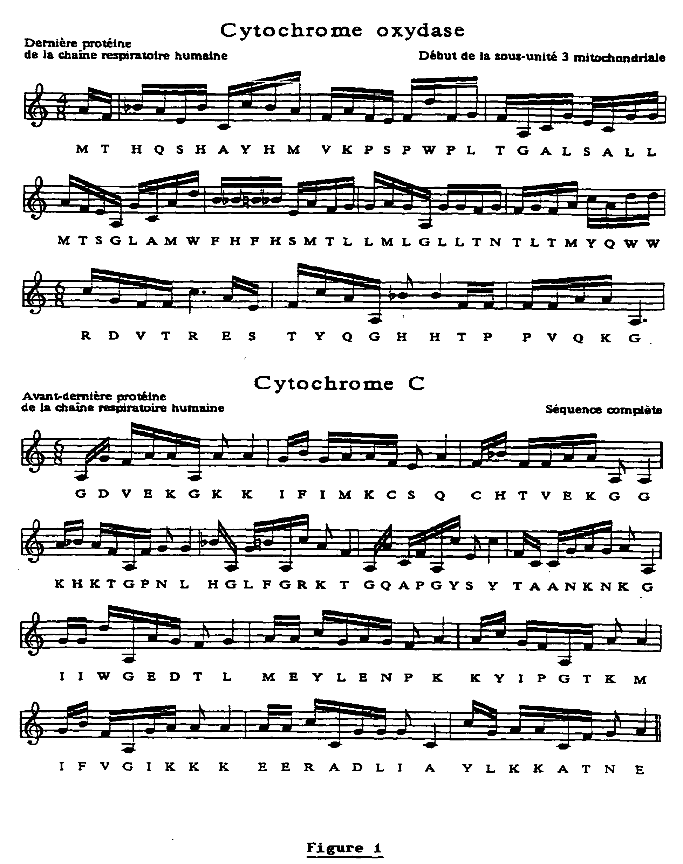

le pic à k = 8 valant environ 2,5 écarts-types (par rapport à sa valeur au hasard 22,3 $\pm$ 4,7 déterminée à partir de la répartition des notes de la molécule); la signification de ce pic étant encore sensiblement renforcée par l'utilisation des distances mélodiques comme décrit en II 2 (elle dépasse nettement 3 écarts-types si l'on inclut les autocorrélations d'intervalles mélodiques, en prenant pour définition de la distance mélodique entre deux notes la valeur absolue de la différence des numéros d'ordre de leurs fréquences tempérées dans la gamme obtenue en II 1, ordonnées dans le sens ascendant -- définition qui dérive de la nomenclature usuelle seconde, tierce, etc... pour les notes d'un mode musical; le pic secondaire à k = 7 devient alors légèrement significatif, correspondant comme nous verrons plus bas à l'allongement relatif de la 7ème note qui tend à précéder le retour sur la tonique, tandis que celui à k = 4 est renforcé lorsqu'on utilise les distances harmoniques comme décrit en II 2, car il correspond, ainsi que nous le verrons plus loin, à des repliements spatiaux de la molécule). L'examen des cadences confirme également cette valeur, de même que celui des homologies internes (ainsi, les cinq dernières notes des premier, deuxième et troisième groupes de 8 forment ensemble une superposition harmonique exacte, autrement dit un canon à trois voix). Plus précisément, ces deux derniers examens amènent à montrer d'abord une plus grande importance relative des septième (cadence F-S sur la deuxième période) et huitième (retour à la tonique, la mineur) notes de chaque période, cette dernière l'emportant encore sur la précédente (la cadence parfaite SQ à la 16ème note l'emportant sur la cadence F-S qui précède, avec le retour à la tonalité initiale); le découpage (le plus économique tenant compte des contraintes précédentes) de la période s'ensuit, six doubles croches, une croche, une noire (à savoir des durées relatives 1-1-1-1-1-1-2-4, avec un rythme en 6:8; cf. figure 1). On notera la cohérence de la progression mélodique (d'où procède principalement la régularité observée) en même temps que la richesse de la progression harmonique, la tonalité de la mineur étant assortie de modulations en mi mineur (mesure 2), sol mineur (mesure 8), fa majeur (mesures 3 et 9) notamment.

Si l'on examine ensuite la distribution des bases de l'ADN, on constate que les première et septième notes de chaque période favorisent, nettement, respectivement l'adénine et la thymine en troisième position, tandis que les troisième et huitième notes favorisent dans les mêmes conditions la cytosine et la guanine. Tout en confirmant le découpage précédent pour la période et les durées relatives des notes (à savoir le fait que les septième et huitième notes ont des durées respectivement double et quadruple de la première), ceci montre de plus que dans un milieu AT-riche, les temps forts vont se trouver sur les première et septième notes, et donc les barres de mesure sur la première, tandis que dans un milieu CG-riche la séquence musicale va démarrer sur une anacrouse (temps forts sur les troisième et huitième notes, barre de mesure sur la troisième). On en conclut que la protéine doit avoir, suivant ces milieux, des rôles métaboliques distincts. De fait, l'étendue de son action métabolique est d'abord attestée par son degré d'évolution musicale (par comparaison avec la séquence de l'Euglène gracile par exemple, par rapport à laquelle, dès les trois premières mesures, on peut observer une amélioration de 56% du niveau [de régularité] mélodique et de 16% du niveau [de régularité] harmonique, définis à partir de la minimisation des distances respectivement mélodiques et harmoniques entre notes successives); la recherche des homologies musicales avec d'autres protéines montre ensuite d'une part une superposabilité avec l'endozepine, avec un cadre de lecture musicale compatible avec la barre de mesure sur la première note, et qui est effectivement une molécule (légèrement) AT-riche: ce qui permet de préciser un rôle "antidépresseur" pour le cytochrome (et sa musique), par désinhibition de la neurotransmission, le cas échéant; et d'autre part un enchaînement musical (en démarrant alors sur une anacrouse) avec la cytochrome oxydase, qui est effectivement (légèrement) CG-riche, et qui termine la chaîne respiratoire, autre rôle métabolique du cytochrome C qui précède dans cette chaîne la cytochrome oxydase.

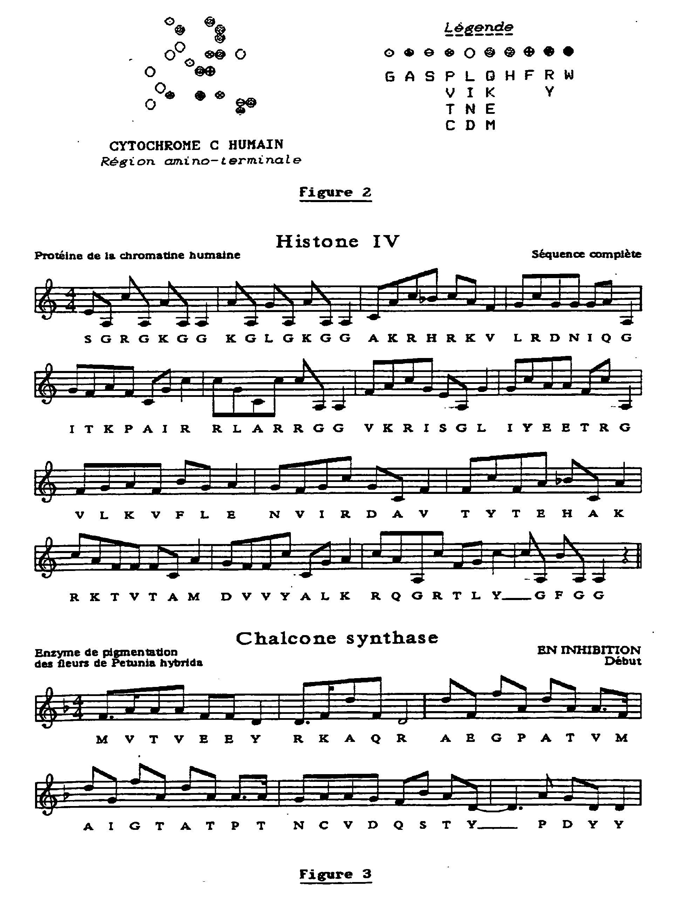

Pour ce qui est du timbre, la tonalité étant ici en la (mineur), la quasi-absence de la quarte (ré) et la faiblesse relative de la quinte (mi) comparée à la nette dominance de la tonique et à l'abondance de l'octave (la grave - la médium) vont privilégier les harmoniques 1 et 2 au détriment des suivants, indiquant un timbre d'orgue, avec en fait des registres un peu différents suivant les passages. Enfin les couleurs se groupent effectivement en "taches" colorées sur la protéine mature (cf. figure 2) avec, comme dans le cas de la musique, de remarquables réponses harmoniques. (A noter que la détermination des couleurs est utile pour confirmer le décodage musical, dans la mesure où certaines autocorrélations de notes se traduisent non dans la période musicale mais dans les repliements spatiaux de la molécule: il faut donc pouvoir le cas échéant les déduire si l'on veut déterminer par ce moyen les périodes musicales; c'est le cas ici ou un "pic" secondaire de ces autocorrélations -- k = 4, dû à l'hélice $\alpha$ du début notamment, que l'on peut voir sur la figure 2 -- correspond à ces repliements. Inversement le décodage musical peut ainsi donner des indications sur la structure spatiale d'une protéine).

2) *Exemple de contrôle du décodage d'une protéine présentant des variations rythmiques.* Comme nous venons de le voir, le décodage d'une protéine peut être contrôlé à plusieurs niveaux, incluant le décodage de molécules connues pour être métaboliquement agonistes, ainsi que la cohérence des prédictions que l'on peut tirer, sur le plan du métabolisme, des homologies musicales observées. On peut de cette façon reconstruire de proche en proche de larges pans du métabolisme à l'échelle moléculaire. Ceci, ainsi que nous allons le voir, facilite à son tour le décodage: dans l'exemple précédent, la "formule rythmique" du cytochrome C peut être schématisée comme suit

```
|6/8 GDVEKGK:K:::| IFIMKCS:Q:::|CHTVEKG:G:::|, etc...
     + + + + + +  + + + + + +   + + + + + +
```

les + soulignant les temps forts et les | indiquant les positions des barres de mesure, tandis que les : représentent l'allongement des notes.

Pour la sous-unité III de la cytochrome oxydase, qui s'enchaîne musicalement avec le cytochrome C, le début est au contraire clairement une formule à quatre temps comme les homologies internes le font voir simplement (ainsi les notes 7 à 22, qui rappellent par leur contour la manière de Bach, se partagent en groupes de quatre notes superposables chacun avec le suivant). A la dixième mesure, on retrouve une mesure non seulement superposable sur tous ses temps forts à la première mesure du cytochrome C, mais en fait même pratiquement identique à la troisième du même cytochrome. Ceci implique un allongement de la mesure, dès la huitième (comme la cadence présente à la fin de cette mesure le signale déjà d'elle-même) en mesure à six temps (fig.1):

```
|4/8 MTHQSHAY|HMVKPSPW|PLTGALSA|LLMTSGLA|
     + + + +  + + + +  + + + +  + + + +
     MWFHFHSM|TLLMLGLL|TNTLTMYQ‖6/8 WWRDVTR:::::|
     + + + +  + + + +  + + + +      + + + + + +
     ESTYQGH:H:::|TPPVQKG:::::‖
     + + + + + + . + + + + + +
```

Ce changement de rythme (de 4/8 en 6/8) est également bien visible dans les autocorrélations de bases de l'ADN, où le pic proéminent passe, à cet endroit, du quatrième au sixième triplet de bases (et bien que le rythme ternaire des bases, qui domine habituellement les autocorrélations de bases des parties codantes de l'ADN, soit ici un peu moins marqué). (Sur la figure 1, on a fait démarrer la séquence sur une anacrouse, accentuant ainsi comme indiqué plus haut le temps fort sur la troisième note, en vue de l'enchaînement avec la variante rythmique en milieu CG-riche du cytochrome C).

3) *Exemple de reconstitution de chaîne métabolique incluant stimulations et inhibitions.* Donnons un autre exemple de reconstitution, de proche en proche, de chaîne métabolique. Le décodage de l'histone 4 est particulièrement aisé: la périodicité de 7 est clairement visible sur la séquence au début de la molécule, les G répétés à deux acides aminés d'intervalle indiquent un rythme binaire, et les cadences G-G qui terminent les deux premières périodes précisent d'emblée un rythme à quatre temps:

```
|SGRGKGG: |KGLGKGG: |;
 + + + +   + + + +
```

ce découpage se poursuit jusqu'à la fin de la séquence, avec la seule exception de la dernière mesure qui est syncopée afin de retrouver par homologie interne la rythmique des deux premières mesures (fig.3). La répartition globale des notes indique une structure harmonique correspondant à un timbre de flûte, et les "sauts de notes" répétés du début, qui suggèrent un son comportant une attaque, permettent même de préciser un timbre de type flûte de pan.

L'histone 4 est une des protéines les plus conservées de tout le règne animal et végétal. Ce qui ne veut pas dire que son action métabolique, manifestement essentielle, n'aie besoin parfois d'être tempérée: ainsi le thème de ses deux premières mesures se retrouve, *en inhibition et transposé d'une quarte,* dans la partie conservée du début de la chalcone synthase, enzyme de pigmentation de nombreuses plantes à fleurs (fig.3). Ceci peut être rapproché du rôle supposé de la chromatine, dont l'histone 4 fait partie, dans la fixation du magnésium: au printemps, les plantes ont besoin de beaucoup de magnésium (pour la photosynthèse) et sa fixation a besoin d'être stimulée (y compris par les chants d'oiseaux qu'évoque le thème de cette molécule); la chalcone synthase est alors inhibée; tandis qu'à l'automne, la stimulation plus faible de l'histone va désinhiber la chalcone synthase et permettre le remplacement du vert des feuilles par les vives couleurs de cette saison, dont on comprend mieux ainsi, de par leur composante épigénétique, la variété tant chantée des poètes.

De fait, en écoutant la transposition sonore de l'histone 4, les auditeurs ont rapporté à plusieurs reprises une "envie de manger du chocolat", qui contient du magnésium (certains ont même signalé "cela fait un peu l'effet du magnésium en granulés, sauf que cet effet est ici immédiat"). Ce qui, peut-on faire remarquer, présente des inconvénients pour des personnes ayant un taux de cholestérol un peu trop élevé. Et de fait, le décodage musical de la chalcone isomérase, métaboliquement agoniste de la chalcone synthase, mais qui "fonctionne mieux" musicalement en stimulation, comporte une suite de thèmes et variations dont la succession chez les plantes à fleurs reproduit les thèmes de toute la chaîne métabolique de régulation du cholestérol *chez l'homme:* l'écoute de cet antagoniste "au deuxième degré" de l'histone 4 permettra ainsi (selon une méthode généralement applicable dans ce type de situation) de corriger éventuellement l'effet secondaire signalé. En outre la fréquence des quartes ascendantes chez la chalcone isomérase tend à se rapprocher de celle que l'on observe chez la myosine chaîne légère alcali des mammifères, qui stimule la contraction musculaire (tandis que le magnésium agit comme on le sait comme décontractant musculaire). Son écoute aura ainsi également pour effet de pousser à l'exercice physique, autre moyen bien connu d'aider la régulation du cholestérol. Ce dernier exemple souligne en fait l'importance d'un phénomène quasi-général, à savoir la coopération épigénétique de différents facteurs dans la stimulation de la synthèse des protéines, qui rend notamment compte de l'aspect sémantique ou informatif par lui-même des séquences musicales: il en est ainsi de l'évocation, par l'écoute de la myosine, de marches militaires par exemple.

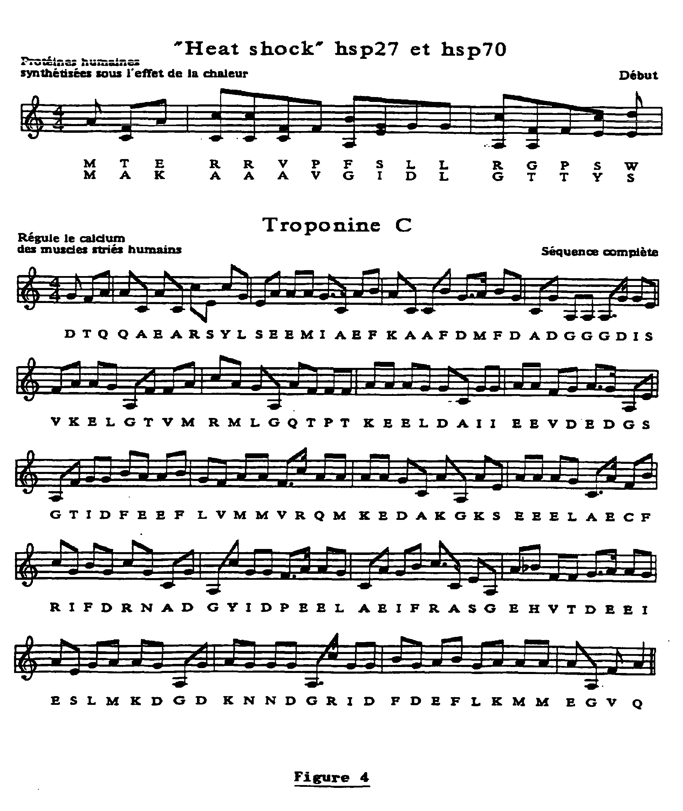

4) Exemple d'analyse biochimique d'une coopération *épigénétique faisant intervenir des superpositions* harmoniques. L'analyse biochimique, lorsqu'elle est possible, de ces coopérations épigénétiques peut encore constituer une aide précieuse au décodage. Ainsi, un autre moyen bien connu de stimuler épigénétiquement la décontraction musculaire est la chaleur, dont l'action bienfaisante sur les rhumatismes par exemple est bien connue. L'action de la chaleur est médiée par un groupe de protéines dites heat *shock,* en général synthétisées ensemble. Ceci suggère que l'on devrait y trouver des superpositions harmoniques: et de fait, la hsp27, qui est apparemment la plus musicale, se superpose avec le début de la hsp70, la plus abondante, qui joue ici un peu le rôle d'une ligne de basse. Ces deux molécules se superposent elles-mêmes avec le début de la troponine C, qui régule le calcium dans la contraction musculaire, et dont on est ainsi amené à prévoir un rôle d'autant plus important comme antirhumatismal que son niveau musical est élevé (fig.4). Il convient cependant de souligner que bien d'autres molécules, elles aussi d'un niveau musical élevé et épigénétiquement sensibles, peuvent se trouver impliquées dans ce type d'affection, depuis la stimulation de la prolactine et de la lipotropine beta (précurseur de la beta-endorphine) jusqu'à l'inhibition, du récepteur de l'oestrogène, en passant par l'inhibition des IgE et de l'interleukine 1 beta.

Ces quelques exemples montrent assez clairement comment de larges pans du métabolisme peuvent ainsi être reconstitués de proche en proche, avec de nombreuses façons de vérifier ou de contrôler la cohérence des résultats obtenus, et de préciser par là-même les décodages musicaux des protéines concernées.

IV. Applications.

Pour les applications, on utilisera les *transcriptions* soit sous forme de partitions musicales, soit sous forme d'enregistrements des séquences musicales obtenues, ainsi que des représentations spatiales colorées des protéines, ensemble ou séparément, sur tout support approprié tel que disque, disquette, cassette audio ou vidéo, ou support papier, tissu ou autre pour les images colorées notamment.

Les enregistrements des séquences musicales peuvent être réalisés à partir des partitions établies comme en II (dont nous avons donné en III quelques exemples), en utilisant l'une des méthodes évaluées dans B. H. Repp, J. Acoust. Soc. Am. 88, p. 622 (1990); la plus précise de ces méthodes ayant été utilisée lors des exemples d'applications présentés ici.

1) Dans les domaines agro-alimentaire et textile d'abord, la possibilité de stimuler certaines synthèses protéiques bien spécifiques, concernant par exemple la lactation des bovins, la fermentation des levures de boulangerie, le goût sucré de certains fruits, ou des fibres animales ou végétales (kératine de la laine des moutons, fibroïne du ver à soie, etc...), ainsi que des protéines propres à certaines plantes officinales; et dans le domaine environnemental, par exemple l'assimilation d'effluents industriels par végétaux interposés, en stimulant la biosynthèse des protéines correspondantes. Nous avons ainsi pu observer, chez une vache à qui étaient donnés à écouter régulièrement pendant 15 jours, au moment de la traite, les enregistrements des transpositions musicales des séquences en acides aminés de la prolactine, de la lactoglobuline et de la lactalbumine bovines, une diminution d'un facteur 3 de la quantité relative de petit lait, résultant en un lait fortement enrichi en protéines et un fromage en conséquence particulièrement savoureux. De même, chez des tomates à qui l'on donnait, pendant leur période de croissance, un "cocktail" de transpositions musicales de protéines diverses incluant des inhibiteurs de virus spécifiques, diverses extensines, puis une enzyme de floraison (la LAT 52), une protéine de défense antibactérienne dont nous attendions également, de par son homologie musicale avec la thaumatine, une amélioration de la teneur en sucre (la P 23), et enfin des inhibiteurs d'enzymes de ramollissement des fruits (pectinesterase et polygalacturonase), nous avons pu observer une nette augmentation de la taille et du nombre des fruits (au total d'un facteur 3,5 environ) en même temps qu'une amélioration sensible du goût sucré chez une proportion significative de celles qui avaient reçu plus particulièrement la P23. Ces résultats remarquables s'assortissent cependant d'un certain nombre de précautions; il existe ainsi des contre-indications à un excès de stimulation de la prolactine notamment, qui doivent être bien prises en compte pour les éleveurs qui effectuent ces opérations, en même temps que chez les animaux eux-mêmes qui peuvent se trouver fragilisés. Ainsi dans les expériences bien connues menées en Israël avec des vaches et des musiques de Mozart -- la prolactine bovine comporte de fait, outre un "niveau musical" particulièrement élevé que l'on peut ici repérer de façon mathématiquement simple (à partir des niveaux mélodique et harmonique, cf. III, § 1), des tournures que l'on peut musicologiquement qualifier de "typiquement mozartiennes" -- le taux de mammites pouvait paraître préoccupant: il convient dans un tel cas (que nous avons également pu observer) de compléter l'écoute de la prolactine par celle de l'alpha-1 antitrypsine, à la musicalité elle aussi très élaborée et au métabolisme complémentaire sur ce point. De même chez les tomates ainsi soumises à des stimuli extérieurs épanouissants pour elles, il faut veiller à ne pas arrêter brutalement en cours de cycle.

Tels quels cependant, ces résultats donnent déjà une indication des ordres de grandeur que l'on peut obtenir dans ce type de conditions, et montrent clairement l'intérêt de l'invention.

2) Dans les domaines thérapeutique et préventif, nombreuses sont les affections se manifestant par une faiblesse métabolique spécifique, et pouvant ainsi être prévenues ou combattues efficacement à l'aide des moyens conformes à la présente invention. La longueur minimale d'une séquence musicalement active étant de l'ordre d'un peptide signal -- de quelques acides aminés à quelques dizainescette action peut être très rapide et apparaître par exemple déjà au bout de quelques secondes ou quelques minutes. Toutefois, l'intégration complète de l'effet produit, métaboliquement complexe, peut prendre davantage de temps, voire nécessiter en cas de fort conditionnement culturel, un certain entraînement initial (correspondant apparemment au "réapprentissage" d'une écoute médiée par les ondes d'échelle provenant du microphonique cochléaire); mais celui-ci s'effectue en général assez rapidement dans l'intérêt manifeste des personnes concernées.

Pour l'emploi responsable du procédé décrit il importe donc de bien connaître le rôle métabolique des molécules impliquées. Or c'est bien sûr l'un des intérêts -- qui dépasse évidemment le seul cadre thérapeutique dont nous parlons ici -- du décodage musical des protéines (ainsi que des couleurs correspondantes) que de permettre, en repérant systématiquement les homologies et anti-homologies musicales (et de couleurs) à partir des séquences protéiques connues et disponibles dans les banques de données, de repérer ainsi les protéines métaboliquement agonistes et antagonistes d'une protéine donnée, dont le degré d'élaboration musicale donne par ailleurs une indication de l'étendue du rôle métabolique. Le procédé décrit permet donc de préciser des indications particulières pour certaines séquences protéiques (comme nous en avons donné plus haut -- en III -- quelques exemples).

Rappelons à ce propos que l'on rencontre fréquemment dans les protéines animales ou végétales, notamment parmi les plus musicales, des fragments mélodiques successifs de chaînes métaboliques humaines, et qu'en conséquence les transpositions actives sur l'homme ne se bornent pas à celles des molécules humaines, comme nous en avons également donné des exemples en III 3. Au contraire le métabolisme de ces espèces apparaissant en quelque sorte comme plus "spécialisé" vers la production de certaines molécules, ce sont bien les protéines "les plus musicales" en général qui seront les plus importantes pour les applications. Bien entendu, ces correspondances entre espèces différentes facilitent encore la délimitation du rôle métabolique, et le décodage même des séquences protéiques.

Il convient d'ailleurs de noter que la musicalité d'une molécule implique en elle-même que sa stimulation épigénétique (en général) est en principe préférable thérapeutiquement, du fait de la portée de ses interactions métaboliques, à son administration directe: les molécules "les plus musicales" sont généralement celles dont soit directement la production par génie génétique, soit l'utilisation thérapeutique qui en résulte rencontre des problèmes, tels que de stabilité, de transport sur le site d'action, ou plus spécifiquement d'effets secondaires liés à des doses devant être beaucoup plus importantes que dans l'organisme pour obtenir des effets comparables, puisque les ondes d'échelle naturellement associées à leur production ne sont alors plus présentes. Ceci est bien sûr particulièrement vrai pour *l'inhibition* des protéines, lorsque l'inhibiteur naturel est par exemple beaucoup plus lourd ou simplement lorsque la production a besoin d'être réduite, à un moment donné (cf. III 3) ou de façon systématique.

Enfin, pour ce qui concerne l'emploi des transcriptions de séquences protéiques, la rapidité même de leur action peut permettre, par comparaisons différentielles, notamment bipolaires, de leur effet positif ou négatif, de préciser laquelle, dans une situation donnée, est la plus appropriée. [A noter que la possibilité ainsi conférée à chacun de se rendre compte par lui-même et très rapidement de l'action de ces transcriptions, et la reconnaissance du soi qui en résulte, ne constituent pas leur moindre intérêt]. Cette identification peut être elle-même facilitée par la comparaison avec les transcriptions de séquences protéiques connues, de phénomènes acoustiques ou électromagnétiques comportant des suites distinctes de fréquences, et dont des effets ont pu être observés dans une situation similaire.

**Revendications**

1. Procédé de régulation épigénétique, soit par stimulation, soit par inhibition de la biosynthèse d'une protéine in situ par résonance d'échelle, **caractérisé en ce que**:

   A. on détermine la séquence d'acides aminés de ladite protéine puis la séquence de notes musicales correspondant à ladite séquence d'acides aminés par décodage et transposition sonore de suites temporelles de vibrations quantiques associées à son élongation en opérant de la manière suivante :

   a) on détermine la fréquence propre de chacun des acides aminés à l'état libre, proportionnelle à sa masse, puis on minimise la distance harmonique globale entre les fréquences de ces acides aminés pour tous les couples d'acides aminés possibles en fonction de leur proportion dans la population environnante des ARN de transfert auxquels lesdits acides aminés se lient en imposant la condition que le déplacement de la fréquence initiale propre à l'état libre déterminée précédemment vers sa valeur à l'état lié (fréquence synchronisée) soit inférieur à la moitié de l'écart entre les deux fréquences synchronisées entourant cette fréquence initiale, puis transposition des fréquences ainsi obtenues dans le domaine audible, ce qui fournit un code permettant la stimulation de la biosynthèse de cette protéine, le codé relatif à son inhibition étant obtenu par symétrisation des logarithmes des fréquences obtenues précédemment par rapport à leur valeur centrale prise comme origine ;
   b) on détermine les périodes musicales par repérage des séquences homologues de notes et de signatures ;
   c) on détermine alors les durées des notes en rectifiant collectivement puis individuellement les périodes déterminées en b) par ajustement du phrasé à la mesure que l'on contrôle à l'aide d'un clavier possédant une touche "one key play";
   d) on détermine le timbre par la rétroaction de l'ensemble des acides aminés de la protéine globale sur la structure harmonique de chacun d'entre eux.

   B. on effectue une diffusion de ladite séquence de notes musicales in situ pour stimuler ou inhiber la biosynthèse de ladite protéine, soit directement, soit indirectement à partir d'enregistrements sur tout support approprié de la séquence de notes musicales obtenues, à l'exclusion du traitement thérapeutique du corps humain ou animal.

2. Procédé selon la revendication 1, **caractérisé en ce que** l'on produit à l'aide d'un instrument convenablement réglé dans ce but, un enchaînement de notes musicales, les notes étant associées aux acides aminés selon le code spécifique à la stimulation épigénétique de la biosynthèse de la protéine suivant la gamme tempérée:
   Gly = la grave ; Ala = do ; Ser = mi ; Pro, Val, Thr, Cys = fa ; Leu, Ile, Asp, Asn = sol; Gln, Glu, Lys, Met = la ; His = si bémol ; Phe (et SeC) = si ; Arg, Tyr = do aigu ; Trp = ré aigu.

3. Procédé selon la revendication 1, **caractérisé en ce que** l'on produit à l'aide d'un instrument convenablement réglé dans ce but, un enchaînement de notes musicales, les notes étant associées aux acides aminés selon le code spécifique à l'inhibition épigénétique de la biosynthèse de la protéine, obtenu en prenant les notes de la gamme tempérée symétriques de celles du code selon la revendication 2 par rapport au sol central :
   Trp = do ; Arg, Tyr = ré ; Phe, SeC = mi bémol ; His = mi ; Gln, Lys, Glu, Met = fa ; Leu, Ile, Asn, Asp = sol ; Pro, Val, Thr, Cys = la ; Ser = si bémol ; Ala = ré aigu ; Gly = fa aigu.

4. Procédé selon la revendication 1 ou la revendication 2, **caractérisé en ce que**, ayant mis en oeuvre ladit procédé, on stabilise la synthèse de ladite protéine à l'aide d'une transposition lumineuse colorée appropriée obtenue en transposant les vibrations quantiques associées à la protéine mature une fois repliée spatialement sur elle-même, selon un code qui se déduit de celui obtenu à l'issue de l'étape a) de la revendication 1 relatif à la stimulation de sa biosynthèse, par application de la formule $\nu \cong \nu_o \mathrm{Argch}(e^{(f/f)\mathrm{Loghcl}})$ où $f, f_o$ sont les fréquences musicales et $\nu, \nu_o$ les fréquences des couleurs, les indices dénotant les valeurs centrales.

5. Procédé selon la revendication 4 **caractérisé en ce que** les positions spatiales des couleurs sont elles qu'occupent les acides aminé dans une réprésentation spatiale tridimensionelle de ladite protéine, le code étant Gly = rouge sombre ; Ala = vermillon ; Ser = orangé ; Pro, Val, Thr, Cys = ocre ; Leu, Ile, Asn, Asp = citron ; Gln, Glu, Lys, Met = vert ; His = émeraude ; Phe = bleu ; Arg, Tyr = indigo ; Trp = violet.

**Claims**

1. A method for epigenetic regulation by stimulating or inhibiting *in situ* biosynthesis of a protein by scale resonance, **characterized by**:

   A. Determining the aminoacid sequence of said protein and then the sequence of musical notes corresponding to said aminoacid sequence by decoding and acoustic transposition of time series of quantum vibrations associated to its elongation by operating in the following way:

      a) determining the proper frequency of each of the aminoacids in its free state, proportional to its mass, then minimizing the global harmonic distance between the frequencies of these aminoacids for all possible pairs of aminoacids taking into account their proportion in the environing population of transfer RNAs to which said aminoacids link while imposing the condition that the displacement of the initial proper frequency for the free state toward its value for the linked state (synchronized frequency) is less than half the interval between the two synchronized frequencies surrounding this initial frequency, then transposing in the audible range the frequencies thus obtained, which provides a code allowing the stimulation of the biosynthesis of said protein, the inhibiting code being obtained by symmetrization of the logarithms of the heretofore obtained frequencies with respect to their central value taken as the origin;
      b) determining the musical periods by spotting homologue sequences of notes and signatures;
      c) then determining the lengths of the notes by rectifying collectively then individually the periods determined in b) by adjusting the phrasing to the measure, which is controlled with the help of a keyboard having a "one key play" key;
      d) determining the tone quality through the retroaction of all the aminoacids of the protein on the harmonic structure of each of them.

   B. *In situ* diffusing said musical notes sequence in order to stimulate or inhibit biosynthesis of said protein, either directly or indirectly from recordings on any appropriate support of the obtained musical notes sequence, with the exclusion of treatment of human or animal body by therapy.

2. A method according to claim 1, **characterized by** producing, with the help of a musical instrument suitably tuned to this end, a chaining of musical notes, the notes being associated to aminoacids according to the specific code for epigenetic stimulation of the biosynthesis of the protein, following the tempered scale:
   Gly = low A; Ala = C; Ser = E; Pro, Val, Thr, Cys = F; Leu, Ile, Asp, Asn = G; Gln, Glu, Lys, Met = A; His = B flat; Phe (and SeC) = B; Arg, Tyr = sharp C; Trp = sharp D.

3. A method according to claim 1, **characterized by** producing, with the help of a musical instrument suitably tuned to this end, a chaining of musical notes, the notes being associated to aminoacids according to the specific code for epigenetic inhibition of the biosynthesis of the protein, obtained by taking the notes of the tempered scale symmetric to those of the code according to claim 2 with respect to the central G:
   Trp = C; Arg, Tyr = D; Phe, SeC = E flat; His = E; Gln, Lys, Glu, Met = F; Leu, Ile, Asn, Asp = G; Pro, Val, Thr, Cys = A; Ser = B flat; Ala = sharp D; Gly = sharp F.

4. A method according to claims 1 or 2, **characterized by**, after performing said method, stabilizing the synthesis of said protein with the help of a suitable colored light transposition obtained by transposing the quantum vibrations associated with the mature protein after it is spatially folded back over itself, according to a code deduced from the code obtained at the end of step a) of claim 1 relative to stimulation of its biosynthesis, by application of the formula

$$v \approx v_0 \, Argch \left( e^{(f/f_0)Logch\, 1} \right)$$

   where $f$, $f_0$ are the musical frequencies and $v$, $v_0$ are the color frequencies, indexes denoting the central values.

5. A method according to claim 4, **characterized in that** the spatial positions of the colors are those occupied by the aminoacids in a spatial three-dimensional representation of the protein, the code being:

Gly = dark red; Ala = bright red; Ser = orange; Pro, Val, Thr, Cys = ochre; Leu, Ile, Asn, Asp = lemon yellow; Gln, Glu, Lys, Met = green; His = emerald; Phe = blue; Arg, Tyr = indigo; Trp = purple.

**Patentansprüche**

1. Verfahren zur epigenetischen Regulation, entweder durch Stimulation oder durch Inhibierung, der Biosynthese eines Proteins *in situ* durch Skalenresonanz, **gekennzeichnet durch**:

   A. Bestimmen der Aminosäuresequenz dieses Proteins und dann der dieser Aminosäuresequenz entsprechenden Sequenz von Musiktönen **durch** Dekodierung und Klangtransposition von zeitlichen Folgen von Quantenvibrationen, die mit seiner Elongation assoziiert sind, wobei in folgender Weise vorgegangen wird:

   a) Bestimmen der Eigenfrequenz von jeder Aminosäure im freien Zustand proportional zu ihrer Masse, anschließendes Minimieren der harmonischen Gesamtdistanz zwischen den Frequenzen dieser Aminosäuren für alle möglichen Aminosäurepaare unter Berücksichtigung ihres Verhältnisses in der Population der umliegenden transfer-RNAs, an die diese Aminosäuren gebunden werden, unter Berücksichtigung der Bedingung, daß die Verschiebung der zuvor bestimmten anfänglichen Eigenfrequenz im freien Zustand zu ihrem Wert im gebundenen Zustand (synchronisierte Frequenz) geringer ist als die Hälfte des Abstands zwischen den beiden diese Anfangsfrequenz einfassenden synchronisierten Frequenzen, anschließende Transposition der so erhaltenen Frequenzen in den hörbaren Bereich, was einen Schlüssel liefert, der die Stimulation der Biosynthese dieses Proteins erlaubt, wobei der Schlüssel zur Inhibierung **durch** Symmetrisierung der Logarithmen der zuvor erhaltenen Frequenzen in Bezug auf ihren als Ausgangspunkt genommenen Zentralwert erhalten wird;
   b) Bestimmen der Dauer der Tonschwingungen **durch** Feststellen der homologen Sequenzen von Tönen und Signaturen;
   c) anschließendes Bestimmen der Länge der Töne **durch** kollektives und dann individuelles Korrigieren der in b) bestimmten Schwingungsdauer **durch** Einstellen der Phrasierung auf die Maßeinheit, was man mit Hilfe eines Keybord kontrolliert, das eine Taste "one key play" besitzt;
   d) Bestimmen der Klangfarbe **durch** die Rückwirkung der Gesamtheit der Aminosäuren des ganzen Proteins auf die harmonische Struktur von jeder von ihnen.

   B. Bewirken einer Ausstrahlung dieser Sequenz von Musiktönen *in situ,* um die Biosynthese des Proteins entweder direkt oder indirekt zu stimulieren, ausgehend von Aufnahmen der erhaltenen Sequenz von Musiktönen auf irgendeinem geeigneten Träger, ausgenommen die therapeutische Behandlung des menschlichen oder tierischen Körpers.

2. Verfahren nach Anspruch 1, **dadurch gekennzeichnet, daß** man mit Hilfe eines zu diesem Zweck entsprechend gestimmten Instruments eine Abfolge von Musiktönen produziert, wobei die Töne mit den Aminosäuren gemäß dem spezifischen Schlüssel für die epigenetische Stimulation der Biosynthese des Proteins in Verbindung gebracht werden, wobei man der temperierten Tonleiter folgt:
   Gly = tiefes A; Ala = C; Ser = E; Pro, Val, Thr, Cys = F; Leu, Ile, Asp, Asn = G; Gln, Glu, Lys, Met = A; His = B, Phe (und SeC) = H; Arg, Tyr = hohes C; Trp = hohes D.

3. Verfahren nach Anspruch 1, **dadurch gekennzeichnet, daß** man mit Hilfe eines zu diesem Zweck entsprechend gestimmten Instruments eine Abfolge von Musiktönen produziert, wobei die Töne mit den Aminosäuren gemäß dem spezifischen Schlüssel für die epigenetische Inhibierung der Biosynthese des Proteins in Verbindung gebracht werden, welcher **dadurch** erhalten wird, daß man die Töne der temperierten Tonleiter nimmt, die zu denen des Schlüssels gemäß Anspruch 2 bezogen auf das zentrale G symmetrisch sind:
   Trp = C; Arg, Tyr = D; Phe, SeC = Es; His = E; Gln, Lys, Glu, Met = F; Leu, Ile, Asn, Asp = G; Pro, Val, Thr, Cys = C; Ser = B; Ala = hohes D; Gly = hohes F.

4. Verfahren nach Anspruch 1 oder Anspruch 2, **dadurch gekennzeichnet, daß** die Synthese des Proteins nach Durchführung des Verfahrens mit Hilfe einer geeigneten Farblichttransposition stabilisiert, die **dadurch** erhalten wird, daß die Quantenschwingungen, die mit dem reifen, einmal räumlich um sich selbst gefalteten Protein assoziiert sind, gemäß einem Schlüssel transponiert werden, der sich von dem ausgehend von Schritt a) von Anspruch 1 zur Stimulation seiner Biosynthese erhaltenen ableitet, und zwar durch Anwendung der Formel

$\nu \approx \nu_0 \text{Arg ch}(e^{(f/f_0)\text{Logch1}})$, wobei $f$, $f_0$ die Tonfrequenzen sind und $\nu$, $\nu_0$ die Frequenzen der Farben sind, wobei die Indizes die Zentralwerte bezeichnen.

5.  Verfahren nach Anspruch 4, **dadurch gekennzeichnet, daß** die Raumpositionen der Farben diejenigen sind, die die Aminosäuren in einer dreidimensionalen räumlichen Darstellung des Proteins einnehmen, mit dem Schlüssel: Gly = dunkelrot; Ala = zinnoberrot; Ser = orange; Pro, Val, Thr, Cys = ockerfarben; Leu, Ile, Asn, Asp = zitronengelb; Gln, Glu, Lys, Met = grün; His = smaragdgrün; Phe = blau; Arg, Tyr = indigo; Trp = violett.

## Cytochrome oxydase

Dernière protéine
de la chaîne respiratoire humaine

Début de la sous-unité 3 mitochondriale

M T H Q S H A Y H M V K P S P W P L T G A L S A L L

M T S G L A M W F H F H S M T L L M L G L L T N T L T M Y Q W W

R D V T R E S T Y Q G H H T P P V Q K G

## Cytochrome C

Avant-dernière protéine
de la chaîne respiratoire humaine

Séquence complète

G D V E K G K K I F I M K C S Q C H T V E K G G

K H K T G P N L H G L F G R K T G Q A P G Y S Y T A A N K N K G

I I W G E D T L M E Y L E N P K K Y I P G T K M

I F V G I K K K E E R A D L I A Y L K K A T N E

**Figure 1**

CYTOCHROME C HUMAIN
*Région amino-terminale*

**Figure 2**

## Histone IV

Protéine de la chromatine humaine — Séquence complète

## Chalcone synthase

Enzyme de pigmentation des fleurs de Petunia hybrida — EN INHIBITION, Début

**Figure 3**

Figure 4

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 8302122

- FR 2541024 A

**Littérature non-brevet citée dans la description**

- **J. STERNHEIMER.** Colloque international ''Louis de Broglie, Physicien et penseur''. *Ancienne Ecole Polytechnique,* 05 Novembre 1987
- **J. STERNHEIMER.** *Ondes d'échelle,* 1992
- **M. O. DAYHOFF.** *Atlas of protein sequence and structure,* 1972, vol. 5
- **P. BUSER ; M. IMBERT.** Audition. 1987

- **Y. HELLEGOUARCH.** *C. R. Math. Rep. Acad. Sci. Canada,* 1982, vol. 4, 277
- **B. ALBERTS et al.** Biologie moléculaire de la cellule. 1990, 539
- **B. H. REPP.** *J. Acoust. Soc. Am.,* 1990, vol. 88, 622